(19) Europäisches Patentamt
European Patent Office
Office européen des brevets

(11) **EP 2 835 662 A1**

(12) **EUROPEAN PATENT APPLICATION**

(43) Date of publication:
**11.02.2015 Bulletin 2015/07**

(51) Int Cl.:
**G01R 33/28** (2006.01)     **G02B 6/35** (2006.01)
**G01R 33/48** (2006.01)

(21) Application number: **14177847.2**

(22) Date of filing: **21.07.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **08.08.2013  KR 20130094216**

(71) Applicant: **Samsung Electronics Co., Ltd Gyeonggi-do 443-742 (KR)**

(72) Inventors:
• **Gi Tae, I**
  **Gyeonggi-do (KR)**
• **Young Dae, Je**
  **Gyeonggi-do (KR)**

(74) Representative: **Land, Addick Adrianus Gosling et al**
**Arnold & Siedsma**
**Bezuidenhoutseweg 57**
**2594 AC The Hague (NL)**

(54) **Signal input apparatus and magnetic resonance imaging apparatus including the same**

(57)     A signal input user interface apparatus comprises an input device including, a plurality of buttons (310), a light waveguide (330), and a plurality of light transmission members (320). The plurality of light transmission members (320) being respectively coupled to the plurality of buttons (310) insertable in the light waveguide (330) to change characteristics of light passing through the light waveguide and in response to activation of an individual button a corresponding respective transmission member is inserted inside the waveguide. A receiver device (400) including a light source is configured to emit light into the light waveguide. A sensor (420) is configured to measure characteristics of light passing through the waveguide including passing through inserted transmission members.

**FIG. 8**

**Description**

A system concerns magnetic resonance imaging to diagnose patient illnesses and identify diseases.

Known medical imaging systems include an X-ray apparatus, an ultrasonic diagnosis apparatus, a computer tomography (CT) apparatus, and a magnetic resonance imaging apparatus. A magnetic resonance imaging apparatus captures images under relatively free conditions and provides excellent soft-tissue contrast and different diagnosis information images valuable for diagnosis. A magnetic resonance imaging (MRI) method may cause a nuclear magnetic resonance phenomenon in hydrogen atomic nuclei in the body using harmless magnetic fields and radio frequency (RF), which is non-ionizing radiation to acquire an image indicating the density and physiochemical characteristics of atomic nuclei. A magnetic resonance imaging apparatus provides a predetermined Radio frequency of particular energy with a magnetic field applied to atomic nuclei, converts energy emitted from the atomic nuclei into a signal, to image the interior of a body.

Since a proton forming an atomic nucleus has a spin angular momentum and a magnetic dipole, when a magnetic field is applied to the proton, the proton is aligned in the direction of the magnetic field, and the atomic nucleus may precess around the direction of the magnetic field. Due to the precessing of the atomic nucleus, an image of the human body may be obtained using a nuclear magnetic resonance phenomenon. Functional MRI (fMRI - hereinafter, referred to as a functional magnetic resonance imaging method) is also known to be used to image functional aspects of the brain and organs. During capture of functional magnetic resonance images, a signal input exclusive of use of electronic components may be employed so that the signal input apparatus can operate in an environment of high magnetic fields. Since electronic components are not used, a signal input apparatus may have a more complicated configuration than when the electronic components are used. As a result, the cost required to embody the signal input apparatus may increase.

A system provides a signal input apparatus capable of inputting signals by means of a plurality of buttons using a single waveguide and a plurality of transmission members having different opacities.

A signal input user interface apparatus comprises an input device including, a plurality of buttons, a light waveguide, and a plurality of light transmission members. The plurality of light transmission members being respectively coupled to the plurality of buttons insertable in the light waveguide to change characteristics of light passing through the light waveguide and in response to activation of an individual button a corresponding respective transmission member is inserted inside the waveguide. A receiver device including a light source is configured to emit light into the light waveguide. A sensor is configured to measure characteristics of light passing through the waveguide including passing through inserted transmission members. The measured characteristics comprise light intensity, light wavelength or frequency.

In a feature, the plurality of transmission members may have different opacities and/or different thicknesses. The transmission members are installed on rear surfaces of the buttons, respectively and are disposed at least partially outside the waveguide before the buttons are activated, and are disposed in a path along which light travels inside the waveguide in response to button activation. Individual transmission members includes reflectors provided on front and rear surfaces to reflect light and reduce light leakage. The light waveguide includes a plurality of openings provided such that the transmission members are located inside the waveguide in response to button activation. A reflector provided on the rear surface of a transmission member is installed to fill a corresponding opening before a corresponding button is activated, and a reflector provided on the front surface of a transmission member is installed to fill the corresponding opening after the corresponding button is activated. An optical fiber is configured to guide light emitted by the light source to the waveguide of the input device and guide light passing through the waveguide of the input device to the sensor of the receiver device. An MRI system is attachable to the input device and the input device operation is unaffected by MRI device magnetic fields.

In another feature, a magnetic resonance imaging apparatus comprises an input device including, a plurality of buttons, a light waveguide, and a plurality of light transmission members respectively coupled to the plurality of buttons and insertable in the light waveguide to change characteristics of light passing through the light waveguide and in response to activation of an individual button a corresponding respective transmission member is inserted inside the waveguide. A receiver device includes a light source configured to emit light into the light waveguide. A sensor is configured to measure characteristics of light passing through the waveguide including passing through inserted transmission members. A workstation is configured to determine which button has been pressed, in response to the measured characteristics. The workstation stores a map associating combinations of buttons and measured light characteristics including at least one of, (a) intensity of light and (b) frequency or wavelength of light. In response to the workstation receiving the data regarding the intensity of light measured by the sensor, the workstation identifies an activated button.

In another feature, a signal input apparatus comprises an input device including a housing, a plurality of buttons exposed outside the housing, a light waveguide provided inside the housing and having at least one surface incorporating a plurality of openings, and a plurality of transmission members installed at a rear surface of individual buttons of the plurality of buttons to fill the plurality of openings and inserted into the light waveguide through the openings in response to button activation. A receiver device includes a light source configured to emit light into the light waveguide. A sensor is configured to measure characteristics of light passing through the waveguide including passing through inserted transmission members. An optical fiber is configured to guide light emitted by the light source to the light waveguide and guide light passing through the waveguide to the sensor.

BRIEF DESCRIPTION OF THE DRAWINGS

These and/or other aspects of the system will become apparent and more readily appreciated from the following description of the embodiments, taken in conjunction with the accompanying drawings of which:

FIG. 1 shows a magnetic resonance imaging apparatus according to invention principles;
FIG. 2 shows an external view of a magnetic resonance imaging apparatus according to invention principles;
FIG. 3 shows a target object space, which is divided by x-, y-, and z-axes according to invention principles;
FIG. 4 shows a bore and a structure of a gradient coil unit according to invention principles;
FIG. 5 shows respective gradient coils of a gradient coil unit and a pulse sequence related with operations of each of the gradient coils according to invention principles;
FIGS. 6 and 7 show a signal input unit for a magnetic resonance imaging apparatus according to invention principles;
FIG. 8 shows construction of a signal input apparatus according to invention principles;
FIG. 9 illustrates buttons, transmission members, and a waveguide of an input device according to invention principles;
FIG. 10 illustrates manipulation of buttons of FIG. 9 and the corresponding transmission member location inside the waveguide according to invention principles;
FIG. 11 shows buttons, transmission members, and a waveguide of an input device according to invention principles;
FIG. 12 shows manipulation of buttons of FIG. 11 and corresponding transmission member location inside the waveguide according to invention principles;
FIG. 13 shows buttons, transmission members, and a waveguide of an input device according to invention principles;
FIG. 14 shows manipulation of buttons of FIG. 13 and corresponding transmission members located inside the waveguide according to invention principles;
FIG. 15 shows buttons, transmission members, and a waveguide of an input device according to invention principles; and
FIG. 16 shows manipulation of buttons of FIG. 15 and corresponding transmission members located inside the waveguide according to invention principles.

DETAILED DESCRIPTION

Reference will now be made in detail to the embodiments of the system, examples of which are illustrated in the accompanying drawings, wherein like reference numerals refer to like elements throughout.

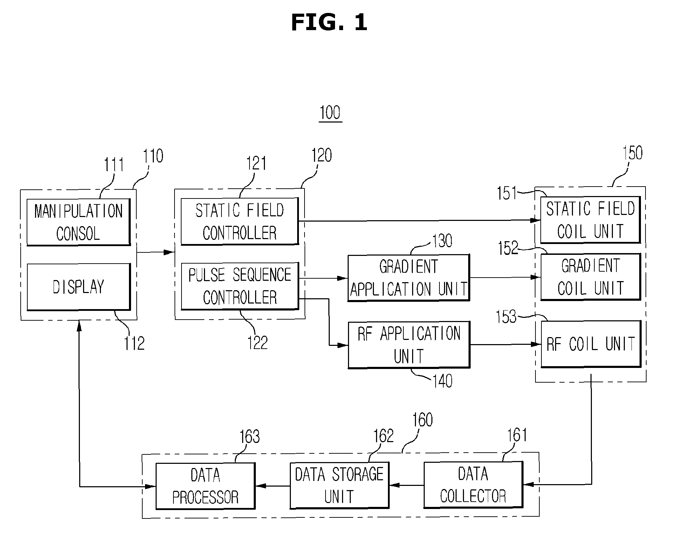

FIG. 1 shows a control block diagram of a magnetic resonance imaging apparatus including a bore 150 configured to form a magnetic field and generate resonance of atomic nuclei, a coil controller 120 configured to control operations of coils constituting the bore 150, an image processor 160 configured to receive an echo-signal generated from the atomic nuclei and generate a magnetic resonance image, and a workstation 110 configured to control overall operations of the magnetic resonance imaging apparatus. The bore 150 may include a static field coil unit 151 configured to form a static field therein, a gradient coil unit 152 configured to form a gradient magnetic field in the static field, and an RF coil unit 153 configured to excite atomic nuclei by applying an RF pulse and receive an echo-signal from the atomic nuclei.

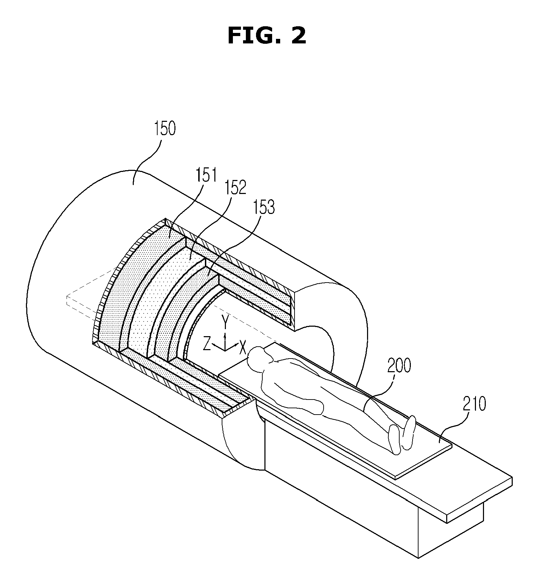

The coil controller 120 may include a static field controller 121 configured to control the intensity and direction of the static field formed by the static field coil unit 151 and a pulse sequence controller 122 configured to provide a pulse sequence and control the gradient coil unit 152 and the RF coil unit 153 according to the selected pulse sequence. The magnetic resonance imaging apparatus may include a gradient application unit 130 configured to apply a gradient signal to the gradient coil unit 152 and an RF application unit 140 configured to apply an RF signal to the RF coil unit 153. The pulse sequence controller 122 may control the gradient application unit 130 and the RF application unit 140 to regulate the gradient magnetic field formed in the static field and RF applied to the atomic nuclei. In addition, the magnetic resonance imaging apparatus may include the workstation 110 so that an operator of the magnetic resonance imaging apparatus can manipulate a system, and receive control commands related with overall operations of the magnetic resonance imaging apparatus 100 from the operator. The workstation 110 may include a manipulation console 111 provided for an operator to manipulate a system and a display unit 112 configured to display a control state, display an image generated by the image processor 160, and enable a user to view an image to diagnose a health state of a target object 200 (FIG. 2).

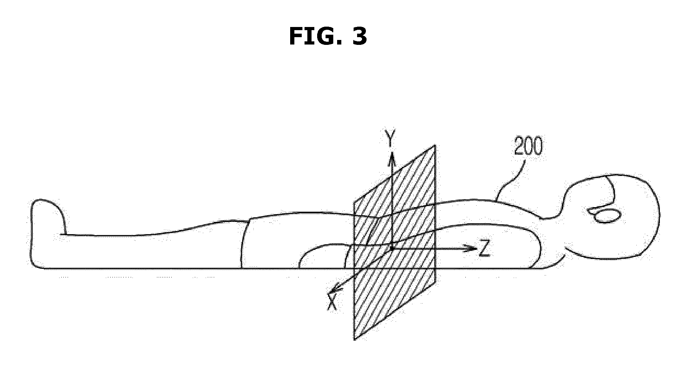

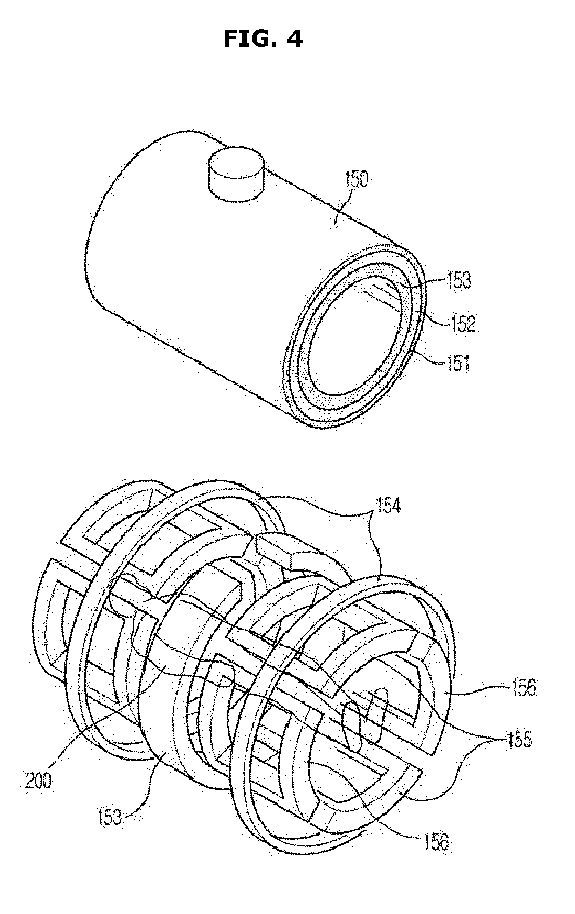

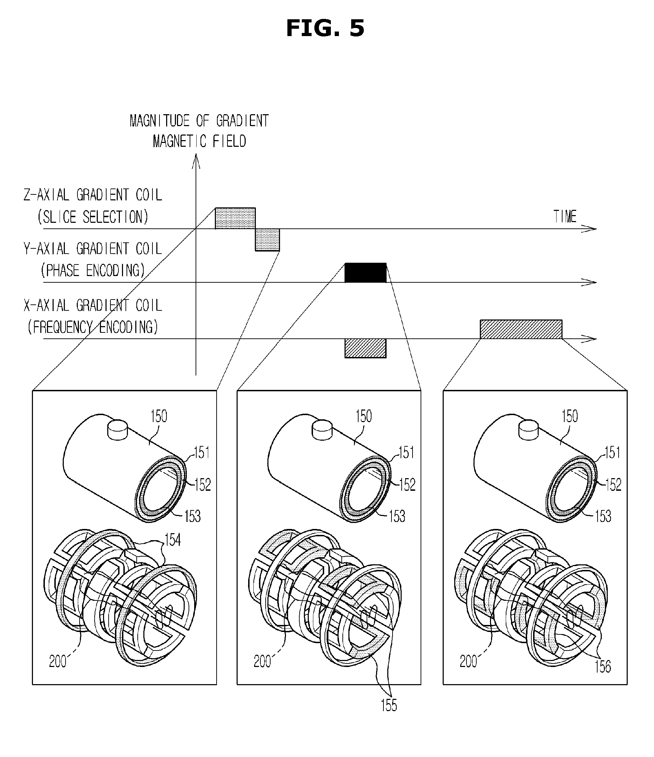

FIG. 2 shows an external view of a magnetic resonance imaging apparatus, and FIG. 3 is a diagram of a target object space, which is oriented by x-, y-, and z-axes. FIG. 4 shows a structure of a bore and a structure of a gradient coil unit, and FIG. 5 shows respective gradient coils of a gradient coil unit and a pulse sequence related with operations of each of the gradient coils. The bore 150 may take on a cylindrical shape having a vacant inner space, and the inner space is referred to as a cavity. A transfer unit may transfer a target object 200 mounted thereon to a cavity unit to obtain a magnetic resonance signal. The bore 150 may include the static field coil unit 151, the gradient coil unit 152, and the RF coil unit 153. The static field coil unit 151 may include a coil wound around the cavity unit. When a current is applied to the static field coil unit 151, a static field may be formed inside the bore 150 (i.e., in the cavity unit).

A direction of the static field may be parallel to the same axis of the bore 150. When a static field is formed in a cavity unit, atoms constituting the target object 200, particularly, atomic nuclei of hydrogen atoms may be aligned in the direction of the static field, and precess around the direction of the static field. A precession speed of the atomic nuclei may be indicated by a precession frequency, which may be referred to as a Larmor frequency and expressed as Equation 1:

$$\omega = \gamma B_0 \quad (1),$$

wherein $\omega$ refers to the Larmor frequency, $\gamma$ refers to a proportional constant, and $B_0$ refers to the intensity of an external magnetic field. The proportional constant $\gamma$ may vary according to the type of atomic nucleus, the unit of the intensity of an external magnetic field is tesla (T) or gauss (G), and the unit of the precession frequency is hertz (Hz). For example, a hydrogen proton has a precession frequency of about 42.58 MHz in an external magnetic field of 1 T. Since hydrogen makes up the largest proportion of atoms constituting the human body, a magnetic resonance imaging apparatus may mainly obtain magnetic resonance signals using the precession of hydrogen protons. The gradient coil unit 152 may generate a gradient in the static field formed in the cavity unit and form a gradient magnetic field.

As shown in FIG. 3, an axis parallel to a direction from the head of the target object 200 to the foot thereof (i.e., an axis parallel to a direction of a static field) is designated a z-axis, an axis parallel to a lateral direction of the target object 200 may be determined as an x-axis, and an axis parallel to a perpendicular direction in the space may be determined as a y-axis. To obtain 3-dimensional spatial information, gradient magnetic fields with respect to all the x-, y-, and z-axes may be needed. Thus, the gradient coil unit 152 may include three pairs of gradient coils.

As shown in FIGS. 4 and 5, a z-axis gradient coil 154 may include a pair of ring-type coils, and a y-axial gradient coil 155 may be disposed on and under the target object 200. An x-axial gradient coil 156 may be disposed on left and right sides of the target object 200. When direct currents having opposite polarities flow in opposite directions from two z-axial gradient coils 154, a variation in a magnetic field may occur in a z-axial direction to form a gradient magnetic field.

FIG. 5 illustrates a pulse sequence in which a z-axial gradient magnetic field is formed during operations of the z-axial gradient coils 154. The z-axial gradient coil 154 may be used to select a slice. As a gradient of the gradient magnetic field formed in the z-axial direction increases, a slice having a smaller thickness may be selected. When the slice is selected using the gradient magnetic field formed by the z-axial gradient coil 154, since spins within the slice have the same frequency and the same phase, the spins cannot be distinguished from one another.

When a magnetic field is formed by the y-axial gradient coil 155 in a y-axial direction, the gradient magnetic field may cause a phase shift so that rows of the slice can have different phases. That is, when a y-axial gradient magnetic field is formed, spins of a row to which a great gradient magnetic field is applied may be phase-shifted to a high frequency, while spins of a row to which a small gradient magnetic field is applied may be phase-shifted to a low frequency. When the y-axial gradient magnetic field disappears, respective rows of a selected slice may be phase-shifted and have different phases so that the rows of the selected slice can be distinguished from each other. Thus, a gradient magnetic field formed by the y-axial gradient coil 155 may be used for phase encoding.

FIG. 5 shows a pulse sequence in which a y-axial gradient magnetic field is formed during operations of the y-axial gradient coil 155. A slice may be selected using a gradient magnetic field formed by the z-axial gradient coil 154, and rows comprising the selected slice selected using the gradient magnetic field formed by the y-axial gradient coil 155 may be distinguished by different phases. However, since spins comprising each row have the same frequency and the same phase, the spins cannot be distinguished from one another. When a gradient magnetic field is formed by the x-axial gradient coil 156 in an x-axial direction, spins comprising each row may have different frequencies due to the gradient magnetic field, and be distinguished from one another. Thus, the gradient magnetic field formed by the x-axial gradient coil 156 may be used for frequency encoding. The gradient magnetic field formed by the z-, y-, and x-axial gradient coils 154, 155, and 156 may undergo a slice selection process, a phase encoding process, and a frequency encoding process and encode spatial positions of the respective spins.

The gradient coil unit 152 may be connected to the gradient application unit 130. The gradient application unit 130 may apply a driving signal to the gradient coil unit 152 and generate a gradient magnetic field in response to a control signal transmitted from the pulse sequence controller 122. The gradient application unit 130 may include three driver circuits corresponding to the three gradient coils 154, 155, and 156 constituting the gradient coil unit 152. Atomic nuclei arranged due to an external magnetic field may precess using a Larmor frequency, and a magnetization vector addition of several atomic nuclei may be indicated by net magnetization M. It may not be possible to measure a z-axis element of the net magnetization M and just x- and y-axial elements of the net magnetization M may be detected. Accordingly, to obtain a magnetic resonance signal, the atomic nuclei are excited so that the net magnetization M can be present on an XY plane. To excite the atomic nuclei, an RF pulse tuned to the Larmor frequency of the atomic nuclei is applied to the static field.

The RF coil unit 153 may include a transmission coil configured to transmit an RF pulse and a receiving coil configured to receive an echo electromagnetic wave (EMW) (i.e., a magnetic resonance signal) emitted by excited atomic nuclei. The RF coil unit 153 may be connected to the RF application unit 140. The RF application unit 140 may apply a driving signal to the RF coil unit 153 and transmit an RF pulse in response to a control signal transmitted from the pulse sequence controller 122. The RF application unit 140 may include a modulation circuit configured to modulate a high frequency output signal into a pulse signal and an RF power amplifier configured to amplify the pulse signal.

In addition, the RF coil unit 153 may be connected to the image processor 160. The image processor 160 may include a data collector 161 configured to receive the magnetic resonance signal received by the RF coil unit 153, process the magnetic resonance signal, and generate data for generating a magnetic resonance image, and a data processor 163 configured to process the data generated by the data collector 161 and generate the magnetic resonance image. The data collector 161 may include a pre-amplifier configured to amplify the magnetic resonance signal received by the receiving coil of the RF coil unit 153, a phase detector configured to receive the magnetic resonance signal from the pre-amplifier and detect a phase of the magnetic resonance signal, and an analog-to-digital (A/D) converter configured to convert an analog signal obtained by detection of the phase into a digital signal. Also, the data collector 161 may transmit the digital-converted magnetic resonance signal to a data storage unit 162.

A data space constituting a 2-dimensional Fourier space may be formed in the data storage unit 162. When storage of the entire data that has completely been scanned is finished, the data processor 163 may transform data stored in a 2-dimensional Fourier space into a 2-dimensional inverse Fourier and reconstruct an image of the target object 200. The reconstructed image may be displayed on the display 112. A spin echo pulse sequence may be mainly used to obtain a magnetic resonance signal from atomic nuclei. During application of an RF pulse from the RF coil unit 153, when a second RF pulse is transmitted after a predetermined time interval since application of a first RF pulse, strong transverse magnetization may occur in atomic nuclei to generate a magnetic resonance signal.

This may be referred to as the spin echo pulse sequence, and a time taken until the magnetic resonance signal is generated after the application of the first RF pulse may be referred to as a time echo (TE). An extent to which a proton is flipped may be indicated by an angle by which the proton moves from an axis on which the proton was located before the proton was flipped. Thus, a 90-degree RF pulse or a 180-degree RF pulse may be expressed according to the flip extent of the proton. A magnetic resonance imaging apparatus is directed to comprehending anatomical structures of the human body and diagnosing disease based on imaged anatomical structures.

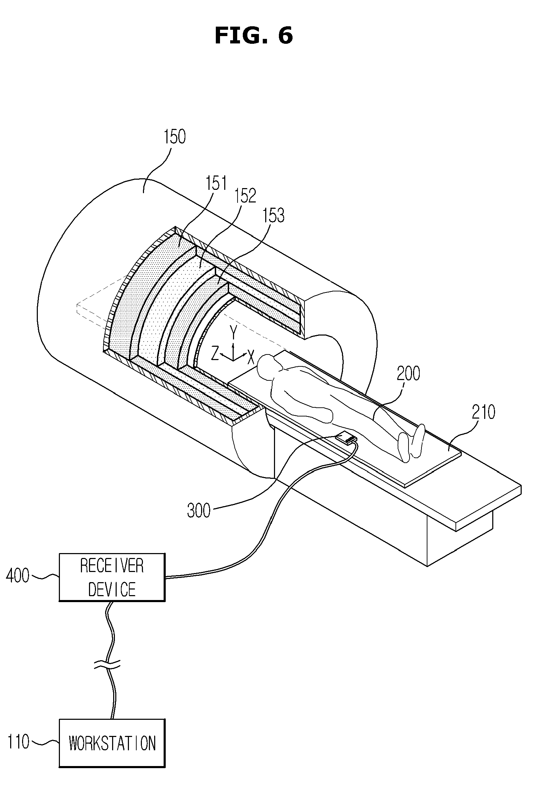

It is known that regions of a brain perform particular corresponding functions, and as brain activity of a specific region increases, the local brain blood flow and metabolism of the specific region increases. A functional magnetic resonance imaging method may include inducing local neural activity in the brain expressing functional positional contrast variation in images. During the imaging of functional magnetic resonance images, a patient may manipulate a signal input apparatus and obtain a functional image of an organ, such as a brain. A signal input apparatus for a magnetic resonance imaging apparatus, which may be manipulated by a patient is described with reference to FIGS. 6 through 16.

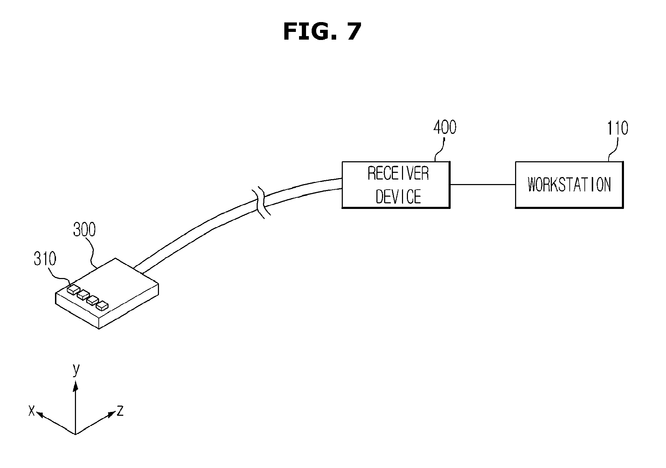

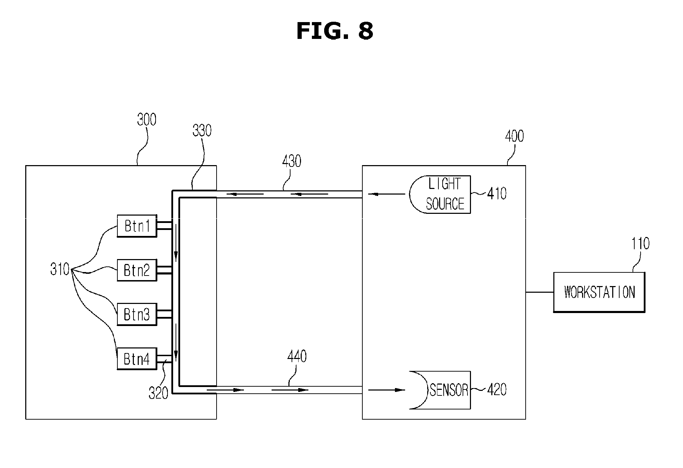

FIGS. 6 and 7 show a signal input apparatus for a magnetic resonance imaging apparatus and FIG. 8 shows construction of a signal input apparatus. The signal input apparatus may include an input device 300 including a structure, such as a button 310 so that a patient can input signals, and a receiver device 400 configured to detect the intensity of light, which may vary according to manipulation of the button 310 of the input device 300.

As shown in FIGS. 6 and 7, input device 300 of the signal input apparatus may include a housing and a plurality of buttons 310. As shown in FIG. 6, a patient may carry the input device 300 so that the patient can manipulate the input device 300 during imaging.

Although FIGS. 6 through 8 illustrate buttons 310 as an example of a structure capable of being manipulated by a patient, different known structures capable of serving the function of the button 310 may also be used. The number of the buttons 310 and the shape or size of the input device 300, may vary. The receiver device 400 may include a light source 410 capable of radiating light to the input device 300 and a sensor 420 capable of measuring the intensity of light received through the input device 300. The receiver device 400 may be connected to the input device 300 by optical fibers 430 and 440. Light output by the light source 410 of the receiver device 400 may be transmitted to the input device 300 through the optical fiber 430, and light passing through the input device 300 may be received by the sensor 420 of the receiver device 400 through the optical fiber 440.

As shown in FIG. 8, the input device 300 may include a plurality of buttons 310 exposed outside the housing and a waveguide 330 by which light output from the light source 410 of the receiver device 400 is transmitted through the optical fiber 430. One end of the waveguide 330 may be connected to the optical fiber 430 configured to guide light output through the light source 410, and the other end of the waveguide 330 may be connected to the optical fiber 440 configured to guide light to the sensor 420. That is, light output by the light source 410 and transmitted through the optical fiber 430 may travel to the waveguide 330 through an inlet of one end of the waveguide 330 to the optical fiber 440 through an outlet of the other end of the waveguide 330. A transmission member 320 may be installed at a rear surface of the button 310, and at one surface of the waveguide 330 so button 310 and the waveguide 330 are connected through the transmission member 320. The transmission member 320 may be formed of opaque material, such as a transmission film, a plastic, a fabric, or an acryl plate. The intensity of light passing through the transmission member 320 is reduced due to opacity of the transmission member 320.

As shown in FIG. 8, transmission members 320 may be respectively installed in buttons 310 installed in the waveguide 330 such that the transmission member 320 provided at the rear surface of the button 310 is located inside the waveguide 330 when a patient manipulates (e.g., presses) button 310. When button 310 is pressed and the transmission member 320 is located inside the waveguide 330, light traveling along the waveguide 330 may pass through the transmission member 320 to reduce the intensity of the light, and the sensor 420 of the receiver device 400 receives and measures the reduced intensity light. The button 310 may be restored to an original state in response to further activation (and pressing) of button 310.

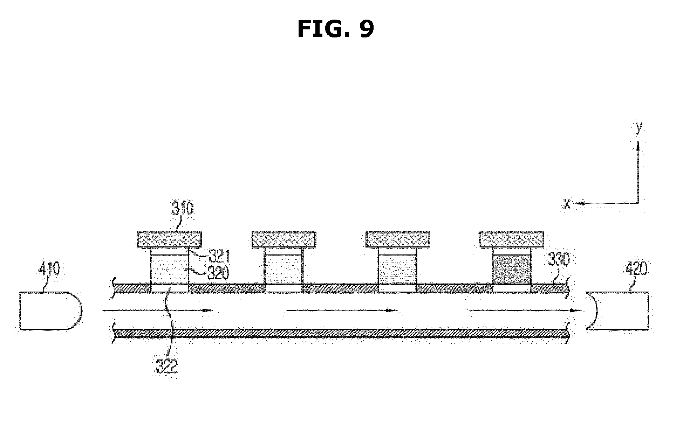

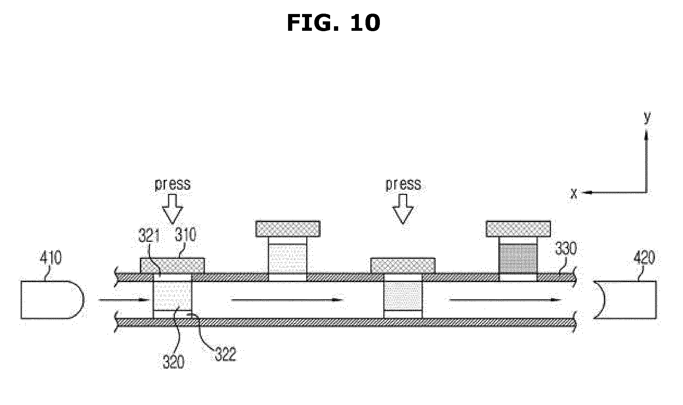

FIG. 9 illustrates buttons 310, transmission members 320, and a waveguide 330 of an input device 300. FIG. 10 illustrates that some of a plurality of buttons 310 are manipulated so that the corresponding transmission members 320 are located inside the waveguide 330. Further transmission members 320 that have different opacities may be respectively installed at rear surfaces of buttons 310. The transmission members 320 may be differently shaded to indicate different opacities of the transmission members 320. For example, the transmission members 320 may be installed such that the opacity of the transmission member 320 increases towards the button 310 installed in a light emission direction and away from the button 310 installed in a light incidence direction. Each of the buttons 310 may be installed on the waveguide 330 in different ways. For instance, openings may be formed in one surface of the waveguide 330 and arranged at a predetermined distance corresponding to a distance between the plurality of buttons 310, and the transmission member 320 installed at the rear surface of the button 310 may be installed in each of the openings to fill the corresponding opening. That is, with button 310 unpressed, the transmission member 320 may be installed to stop the opening of the waveguide 330. The opening may be formed to have the same size and shape as an x-axial section of the transmission member 320 so that light traveling along the waveguide 330 cannot leak out through the opening. In addition, to precisely prevent light traveling along the waveguide 330 from leaking out through the opening, a second reflector 322 capable of reflecting incident light may be installed at the rear surface of each of the transmission members 320.

The second reflector 322 may stop the opening of the waveguide 330 and reflect light incident to the opening so that light can be better prevented from leaking out. When light leaks out through the opening, the intensity of light measured by a sensor 420 may be reduced more than light intensity reduced by the transmission member 320. As a result, the precision of measurement may be degraded. Therefore, the precision of measurement may be improved by preventing light from leaking out using the second reflector 322. Furthermore, a first reflector 321 may be installed at the front surface of each of the transmission members 320. That is, as shown in FIG. 9, the first reflector 321 may be installed between the transmission member 320 and the button 310. When the button 310 is pressed and the transmission member 320 is located inside the waveguide 330, the second reflector 322 installed at the rear surface of the transmission member 320 to stop the opening of the waveguide 330 may be located inside the waveguide 330 along with the transmission member 320. Also, the first reflector 321 installed at the front surface of the transmission member 320 may stop the opening of the waveguide 330, like the second reflector 322 installed at the rear surface of the transmission member 320 before the button 310 is pressed. Therefore, before and after the button 310 is pressed, the opening of the waveguide 330 may be stopped by the reflectors 321 and 322 so that light can be prevented from leaking out.

Specifications of an assembly including the transmission member 320 and the first and second reflectors 321 and 322 may be determined based on the inner diameter of the waveguide 330 so that the second reflector 322 may stop the opening of the waveguide 330 before the button 310 is pressed, and the first reflector 321 may stop the opening of the waveguide 330 after the button 310 is pressed. As shown in FIG. 10, when the button 310 is pressed, the transmission member 320 may be located on a path along which light travels inside the waveguide 330. While light is passing through the transmission member 320, the intensity of the light may be reduced according to the opacity of the transmission member 320. The opacity of transmission members 320 is selected so that if all the transmission members 320 are located on the path along which light travels in the waveguide 330, light passing through the waveguide 330 is still detectable by a sensor 420. Further, the opacities of transmission members 320 are known so it is determined which buttons 310 (including 1 to all) have been pressed based on the measured intensity of light detected by the sensor 420.

A combination of the buttons 310 may be determined, and the intensity of light measured when the determined combination of buttons 310 is pressed. Thus, it may be determined which one or ones of the buttons 310 have been pressed, based on the intensity of light measured by the sensor 420. Combinations of the buttons 310 and data regarding the intensities of light according thereto may be previously stored in the workstation 110. When the sensor 420 of the receiver device 400 measures the intensity of light and outputs the measured intensity of light to the workstation 110, the workstation 110 may determine which one of the buttons 310 has been pressed, based on the previously stored data.

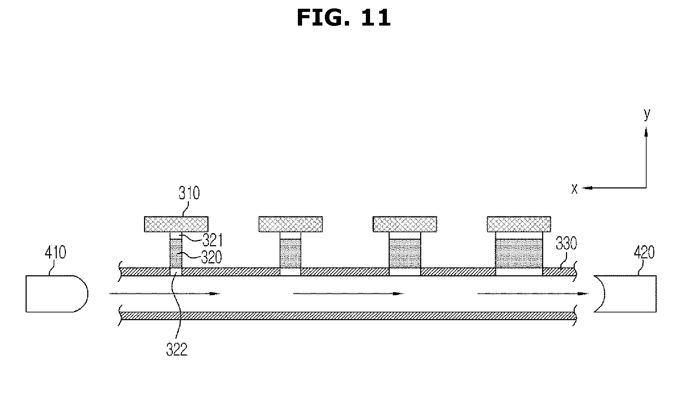

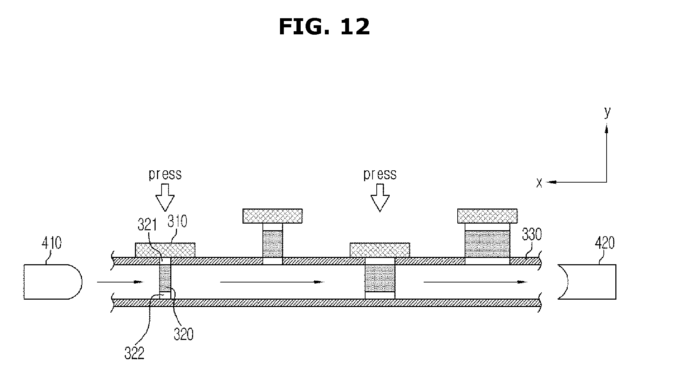

FIG. 11 shows buttons 310, transmission members 320, and a waveguide 330 of an input device 300. FIG. 12 shows that some of a plurality of buttons 310 are manipulated so that the corresponding transmission members 320 are located inside the waveguide 330. As shown in fig. 11 transmission members 320 having different x-axial thicknesses are respectively installed at rear surfaces of four buttons 310. For example, the transmission members 320 may be installed such that the x-axial thickness of the transmission member 320 increases towards the button 310 installed in a light emission direction and away from the button 310 installed in a light incidence direction. Each of the buttons 310 may be installed on the waveguide 330 in different ways. For instance, openings may be formed in one surface of the waveguide 330 and arranged at a predetermined distance corresponding to a distance between the plurality of buttons 310. The transmission member 320 installed at the rear surface of the button 310 may be installed in the opening of the waveguide 330 to stop the opening thereof. That is, with the button 310 unpressed, the transmission member 320 may be installed to stop the opening of the waveguide 330. Since the transmission members 320 have different thicknesses, the openings formed in the waveguide 330 may be provided to have different sizes corresponding to the thicknesses of the transmission members 320. The opening may be formed to have the same size and shape as an x-axial section of the transmission member 320 so that light traveling along the waveguide 330 cannot leak out through the opening of the waveguide 330.

In addition, to precisely prevent light traveling along the waveguide 330 from leaking out through the opening, a second reflector 322 capable of reflecting incident light may be installed at the rear surface of each of the transmission members 320.

The second reflector 322 installed at the rear surface of the transmission member 320 may stop the opening of the waveguide 330 and reflect light incident to the opening so that light can be better prevented from leaking out. When light leaks out through the opening, the intensity of light measured by a sensor 420 may be reduced more than light intensity reduced by the transmission member 320. As a result, the precision of measurement may be degraded. Furthermore, a first reflector 321 may be installed at the front surface of each of the transmission members 320 between the transmission member 320 and the button 310. When the button 310 is pressed and the transmission member 320 is located inside the waveguide 330, the second reflector 322 installed at the rear surface of the transmission member 320 to stop the opening of the waveguide 330 may be located inside the waveguide 330 along with the transmission member 320. Also, the first reflector 321 installed at the front surface of the transmission member 320 may stop the opening of the waveguide 330, like the second reflector 322 installed at the rear surface of the transmission member 320 before the button 310 is pressed. Therefore, before and after the button 310 is pressed, the opening of the waveguide 330 may be stopped by the reflectors 321 and 322 so that light can be prevented from leaking out.

Specifications of an assembly including the transmission member 320 and the first and second reflectors 321 and 322 may be determined in response to the inner diameter of the waveguide 330 so that the second reflector 322 may stop the opening of the waveguide 330 before the button 310 is pressed, and the first reflector 321 may stop the opening of the waveguide 330 after the button 310 is pressed. Unlike in FIGS. 9 and 10, since a plurality of transmission members 320 have different thicknesses, specifications of the first and second reflectors 321 and 322 installed at the front and rear surfaces of each of the transmission members 320 may be differently determined according to each button 310.

As shown in FIG. 12, when the button 310 is pressed, the transmission member 320 may be located on a path along which light travels inside the waveguide 330. While light is passing through the transmission member 320, the intensity of the light may be reduced according to the thickness of the transmission member 320. When light passes through a third transmission member 320, the intensity of light may be reduced more than when light passes through a first transmission member 320 provided in a direction in which light is incident. For example, as shown in FIG. 12, when a transmission member 320 of a button 310 is set to be twice as thick as a transmission member 320 of the preceding button 310, the intensity of light reduced when light passes through the third transmission member 320 may be four times the intensity of light reduced when light passes through the first transmission member 320. If all the transmission members 320 are located on the path along which light travels in the waveguide 330, light passing through the waveguide 330 should have such an intensity that the light can be detected by a sensor 420. The thickness of the transmission member 320 may be previously determined in consideration of the above-described point. The thicknesses of the transmission members 320 are known so it may be determined which one or ones of buttons 310 have been pressed based on the intensity of light detected by the sensor 420.

The intensity of light measured when a combination of buttons 310 is pressed is used to determine a combination of the buttons 310 that are pressed. Thus, it may be determined which one of the buttons 310 has been pressed, based on the intensity of light measured by the sensor 420. Combinations of the buttons 310 and data regarding the intensities of light comprising a map associating the different combinations of pressed buttons with intensity of light expected, is stored in the workstation 110. When the sensor 420 of the receiver device 400 measures the intensity of light and outputs the measured intensity of light to the workstation 110, the workstation 110 uses the map to determine which one of the buttons 310 has been pressed, based on the previously stored data.

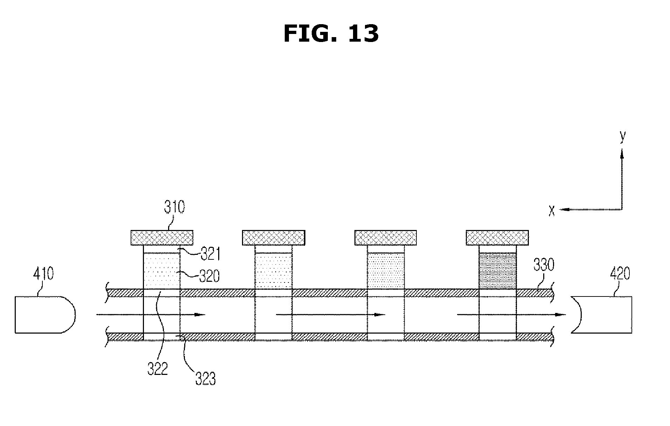

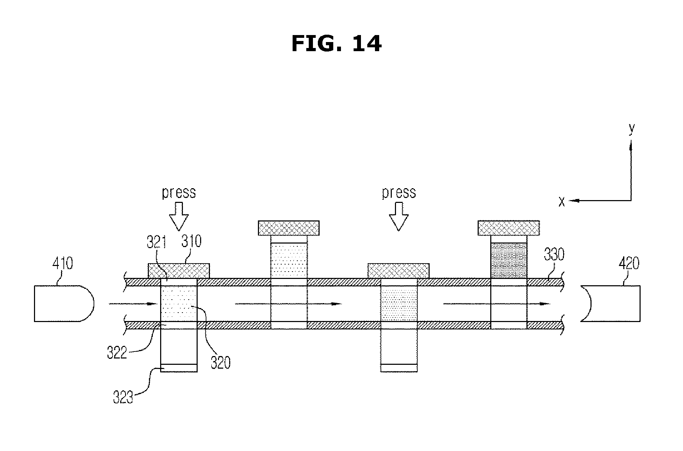

FIG. 13 shows buttons 310, transmission members 320, and a waveguide 330 of an input device 300. FIG. 14 shows some of a plurality of buttons 310 are manipulated so that the corresponding transmission members 320 are located inside the waveguide 330. Referring to FIG. 13, transmission members 320 having different opacities are respectively installed at rear surfaces of four buttons 310. The transmission members 320 may be differently shaded to indicate different opacities of the transmission members 320. For example, the transmission members 320 may be installed such that the opacity of the transmission member 320 increases towards a light emission direction and away from the light incidence direction. Each of the buttons 310 may be installed on the waveguide 330 in different ways. For instance, front openings may be formed in one surface (hereinafter, referred to as a front surface) of the waveguide 330 disposed adjacent to the buttons 310 and arranged at a predetermined distance corresponding to a distance between the plurality of buttons 310, and rear openings having the same shape may be further formed in positions corresponding to the front openings in a surface (hereinafter, referred to as a rear surface) of the waveguide 330, which may face the surface in which the front openings are formed. That is, through holes may be formed through the front and rear openings formed in the opposite front and rear surfaces of the waveguide 330, and provided in equal number to the number of the buttons 310 in a y-axial direction.

The transmission member 320 installed at the rear surface of the button 310 may be installed in the front opening of the waveguide 330 to fill the front opening and a third reflector 323 may be installed in the rear opening facing the front opening to fill the rear opening. That is, with the button 310 unpressed, the transmission member 320 may be installed to fill the front opening of the waveguide 330, and the third reflector 323 may be installed to fill the rear opening of the waveguide 330. The openings may be formed to have the same sizes and shapes as an x-axial section of the transmission member 320 and the third reflector 323 so that light traveling along the waveguide 330 cannot leak out through the front and rear openings. In addition, to precisely prevent light traveling along the waveguide 330 from leaking out through the front opening, a second reflector 322 capable of reflecting incident light may be installed at the rear surface of each of the transmission members 320 as shown in FIG. 13.

The third reflector 323 installed at the rear opening of the waveguide 330 may fill the rear opening of the waveguide 330, and the second reflector 322 installed at the rear surface of the transmission member 320 may fill the front opening of the waveguide 330 so that light incident at the openings can be better prevented from leaking out. As shown in FIG. 13, the first reflector 321 may be installed between the transmission member 320 and the button 310. When the button 310 is pressed and the transmission member 320 is located inside the waveguide 330, as shown in FIG. 14, the third reflector 323, which has filled the rear opening of the waveguide 330, may move out of the waveguide 330. Also, the second reflector 322 installed at the rear surface of the transmission member 320 to fill the front opening of the waveguide 330 may be located in the rear opening of the waveguide 330 to fill the rear opening thereof. Also, the first reflector 321 installed at the front surface of the transmission member 320 may fill the front opening of the waveguide 330, like the second reflector 322 installed at the rear surface of the transmission member 320 before the button 310 is pressed. Therefore, before and after the button 310 is pressed, the front and rear openings of the waveguide 330 may be respectively filled by the reflectors 322 and 323 so that light can be prevented from leaking out.

As shown in FIGS. 13 and 14, a button (310) assembly including the button 310, the transmission member 320, and the first, second, and third reflectors 321, 322, and 323 may be installed in the waveguide 330 in a shape inserted into the through hole formed through the front and rear openings of the waveguide 330. When the button 310 is pressed, as described above, the button (310) assembly may move through the through hole in a rear-surface direction such that the transmission member 320 is located inside the waveguide 330.

Specifications of the button (310) assembly may be determined in response to the inner diameter of the waveguide 330 so that the second reflector 322 and the third reflector 323 may respectively fill the front and rear openings of the waveguide 330 before the button 310 is pressed, and the first reflector 321 and the second reflector 322 may respectively fill the front and rear openings of the waveguide 330 after the button 310 is pressed.

As shown in FIG. 14, when the button 310 is pressed, the transmission member 320 may be located on a path along which light travels inside the waveguide 330. While light is passing through the transmission member 320, the intensity of the light may be reduced according to the opacity of the transmission member 320. If all the transmission members 320 are located on the path along which light travels in the waveguide 330, light passing through the waveguide 330 has a measured intensity detected by a sensor 420 to detect any combination of pressed buttons 310.

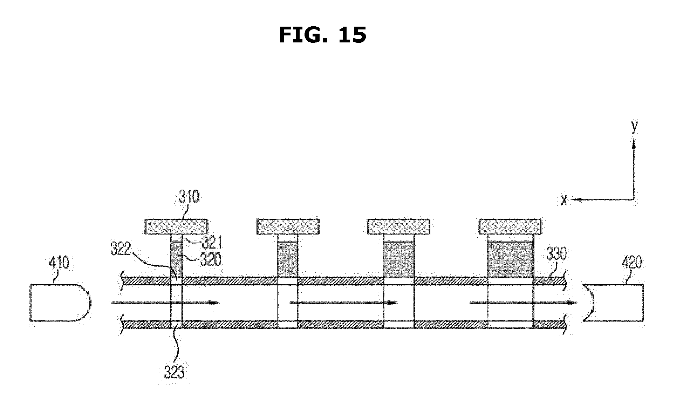

As shown in FIG. 15, transmission members 320 having different x-axial thicknesses are respectively installed at rear surfaces of four buttons 310. For example, the transmission members 320 may be installed such that the x-axial thickness of the transmission members 320 increase towards a light emission direction and away from a light incidence direction. Buttons 310 may be installed on the waveguide 330 in different ways. For instance, front openings may be formed in a front surface of the waveguide 330 and spaced at a predetermined distance and rear openings having the same shape may be further formed in positions corresponding to the front openings in a rear surface of the waveguide 330. That is, through holes may be formed through the front and rear openings formed in the opposite front and rear surfaces of the waveguide 330, and provided in equal number to the number of the buttons 310 in a y-axial direction.

The transmission member 320 installed at the rear surface of the button 310 may be installed in the front opening of the waveguide 330 to fill the front opening thereof, and a third reflector 323 may be installed in the rear opening facing the front opening to fill the rear opening. Since the transmission members 320 have different thicknesses, the front and rear openings formed in the waveguide 330 may be provided to have different sizes corresponding to the thicknesses of the transmission members 320. The openings may be formed to have the same sizes and shapes as an x-axial section of the transmission member 320 using third reflector 323 to prevent light leakage. A first reflector 321 and second reflector 322 similarly prevents light leakage.

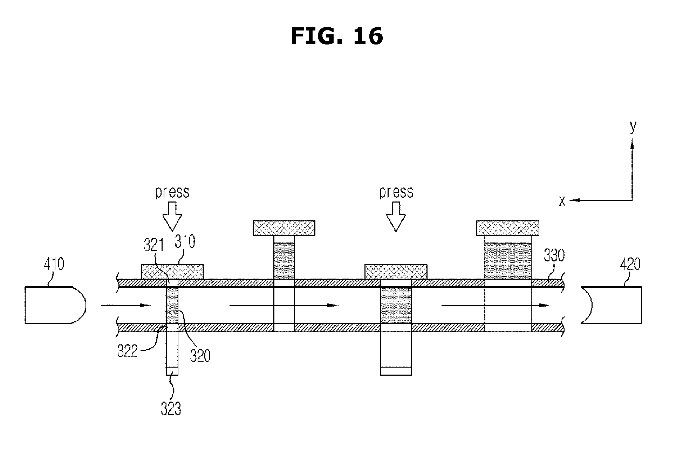

Furthermore, as shown in FIG. 15, the first reflector 321 may be installed between the transmission member 320 and the button 310. When the button 310 is pressed and the transmission member 320 is located inside the waveguide 330, as shown in FIG. 16, the third reflector 323, which has filled the rear opening of the waveguide 330, may move out of the waveguide 330. Also, the second reflector 322 installed at the rear surface of the transmission member 320 to fill the front opening of the waveguide 330 may be located to fill the rear opening. Also, the first reflector 321 installed at the front surface of the transmission member 320 may fill the front opening of the waveguide 330, like the second reflector 322 installed at the rear surface of the transmission member 320 before the button 310 is pressed. Therefore, before and after the button 310 is pressed, the front and rear openings of the waveguide 330 may be respectively filled by the reflectors 322 and 323 to prevent light leakage. Unlike in FIGS. 13 and 14, since a plurality of transmission members 320 have different thicknesses, specifications of the first, second, and third reflectors 321, 322, and 323 may be differently determined according to each button 310.

As shown in FIG. 16, when light passes through a third transmission member, the intensity of light may be reduced more than when light passes through a first transmission member (ordered in direction of light travel). For example, as shown in FIG. 16, when a transmission member is set to be twice as thick as a transmission member of a preceding button, the intensity of light is reduced four times as much as when light passes through the first transmission member, for example. Sensor 420 detects all the transmission members located in the path of light travel. The system detects all combinations of button presses based on known light reduction characteristics of the different transmission members and associated thicknesses.

A combination of the buttons 310 may be determined, and the intensity of light measured when the determined combination of buttons 310 is pressed. Thus, it may be determined which one of the buttons 310 has been pressed, based on the intensity of light measured by the sensor 420. Combinations of the buttons 310 and data regarding the intensities of light is stored in the workstation 110. When the sensor 420 of the receiver device 400 measures the intensity of light and outputs the measured intensity of light to the workstation 110, the workstation 110 may determine which one of the buttons 310 has been pressed, based on the previously stored data.

In a further embodiment, transmission members 320 individually filter and exclude different light wavelengths and sensor 420 detects individual presses based on the excluded light wavelengths.

As is apparent from the above description, configuration of an apparatus can be simplified using a small number of optical fibers, and cost required to constitute the apparatus can be reduced. It would be appreciated by those skilled in the art that changes may be made in these embodiments without departing from the principles herein as defined in the claims and their equivalents.

The above-described embodiments can be implemented in hardware, firmware or via the execution of software or computer code that can be stored in a recording medium such as a CD ROM, a Digital Versatile Disc (DVD), a magnetic tape, a RAM, a floppy disk, a hard disk, or a magneto-optical disk or computer code downloaded over a network originally stored on a remote recording medium or a non-transitory machine readable medium and to be stored on a local recording medium, so that the methods described herein can be rendered via such software that is stored on the recording medium using a general purpose computer, or a special processor or in programmable or dedicated hardware, such as an ASIC or FPGA. As would be understood in the art, the computer, the processor, microprocessor controller or the programmable hardware include memory components, e.g., RAM, ROM, Flash, etc. that may store or receive software or computer code that when accessed and executed by the computer, processor or hardware implement the processing methods described herein. In addition, it would be recognized that when a general purpose computer accesses code for implementing the processing shown herein, the execution of the code transforms the general purpose computer into a special purpose computer for executing the processing shown herein. The functions and process steps herein may be performed automatically or wholly or partially in response to user command. An activity (including a step) performed automatically is performed in response to executable instruction or device operation without user direct initiation of the activity. No claim element herein is to be construed under the provisions of 35 U.S.C. 112, sixth paragraph, unless the element is expressly recited using the phrase "means for."

**Claims**

1. A signal input user interface apparatus **characterized by**:

    an input device including,

    a plurality of buttons (310),
    a light waveguide (330), and
    a plurality of light transmission members (320) respectively coupled to the plurality of buttons and selectively insertable in the light waveguide to change characteristics of light passing through the light waveguide and in response to activation of an individual button a corresponding respective transmission member is inserted inside the waveguide;

    a receiver device (400) including a light source configured to emit light into the light waveguide; and
    a sensor (420) configured to measure characteristics of light passing through the waveguide including passing through selectively inserted transmission members.

2. The apparatus according to claim 1, **characterized in that** the measured characteristics comprise at least one of, light intensity, light wavelength or frequency.

3. The apparatus according to claim 1 or 2, **characterized in that** the plurality of transmission members are provided to have different opacities.

4. The apparatus according to claim 1, 2 or 3, **characterized in that** the plurality of transmission members are provided to have different thicknesses.

5. The apparatus according to any of claims 1-4, **characterized in that** the transmission members are installed on rear surfaces of the buttons, respectively.

6. The apparatus according to any of claims 1-5, **characterized in that** the transmission members are disposed at least partially outside the waveguide before the buttons are activated, and are disposed in a path along which light travels inside the waveguide in response to button activation.

7. The apparatus according to any of claims 1-6, **characterized in that** individual transmission members includes reflectors provided on front and rear surfaces to reflect light and reduce light leakage.

8. The apparatus according to claim 7, **characterized in that** the waveguide includes a plurality of openings provided such that the transmission members are located inside the waveguide in response to button activation.

9. The apparatus according to claim 8, **characterized in that** a reflector provided on the rear surface of a transmission member is installed to fill a corresponding opening before a corresponding button is activated, and a reflector provided on the front surface of a transmission member is installed to fill the corresponding opening after the corresponding button is activated.

10. The apparatus according to any of claims 1-9, **characterized by** an optical fiber configured to guide light emitted by the light source to the waveguide of the input device and guide light passing through the waveguide of the input device to the sensor of the receiver device.

11. A magnetic resonance imaging apparatus **characterized by**:

    an input device including,

    a plurality of buttons (310),
    a light waveguide (330), and
    a plurality of light transmission members (320) respectively coupled to the plurality of buttons and selectively insertable in the light waveguide to change characteristics of light passing through the light waveguide and in response to activation of an individual button a corresponding respective transmission member is inserted inside the waveguide;

    a receiver device (400) including a light source configured to emit light into the light waveguide;
    a sensor (420) configured to measure characteristics of light passing through the waveguide including passing through selectively inserted transmission members; and
    a workstation (110) configured to determine which button has been pressed, in response to the measured characteristics.

12. The apparatus according to claim 11, **characterized in that** the workstation stores a map associating combinations of buttons and measured light characteristics including at least one of, (a) intensity of light and (b) frequency or wavelength of light.

13. A signal input apparatus **characterized by**:

an input device including a housing, a plurality of buttons (310) exposed outside the housing, a light waveguide (330) provided inside the housing and having at least one surface incorporating a plurality of openings, and a plurality of transmission members installed at a rear surface of individual buttons of the plurality of buttons to fill the plurality of openings and inserted into the light waveguide through the openings in response to button activation;
a receiver device (400) including a light source configured to emit light into the light waveguide; and
a sensor (420) configured to measure characteristics of light passing through the waveguide including passing through selectively inserted transmission members; and
an optical fiber (430, 440) configured to guide light emitted by the light source to the light waveguide and guide light passing through the waveguide to the sensor.

# FIG. 1

100

| 111 110 | 121 120 | | 150 |
| MANIPULATION CONSOL | STATIC FIELD CONTROLLER | 151 | STATIC FIELD COIL UNIT |
| DISPLAY | PULSE SEQUENCE CONTROLLER | 130 GRADIENT APPLICATION UNIT | 152 GRADIENT COIL UNIT |
| 112 | 122 | RF APPLICATION UNIT 140 | 153 RF COIL UNIT |
| | 163 DATA PROCESSOR | 162 160 DATA STORAGE UNIT | 161 DATA COLLECTOR |

# FIG. 2

# FIG. 3

# FIG. 4

# FIG. 5

# FIG. 6

# FIG. 7

# FIG. 8

# FIG. 9

# FIG. 10

# FIG. 11

# FIG. 12

**FIG. 13**

# FIG. 14

# FIG. 15

# FIG. 16

**EUROPEAN SEARCH REPORT**

Europäisches Patentamt
European Patent Office
Office européen des brevets

Application Number

EP 14 17 7847

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | GB 2 295 687 A (FORD MOTOR CO [GB])<br>5 June 1996 (1996-06-05)<br>* page 2, line 17 - line 28 *<br>* page 4, line 21 - page 5, line 14;<br>claims 1-4,10; figures 1,2 *<br>----- | 1-3,5,6,<br>10,13<br>4,7-9 | INV.<br>G01R33/28<br>G02B6/35<br><br>ADD.<br>G01R33/48 |
| Y | US 7 039 266 B1 (DOTY MICHAEL [US])<br>2 May 2006 (2006-05-02)<br>* column 1, line 8 - line 14 *<br>* column 4, line 62 - column 6, line 36;<br>claims 1-3 *<br>----- | 11,12 | |
| X<br>Y<br>A | DE 35 11 020 A1 (SINGER CO [US])<br>3 October 1985 (1985-10-03)<br>* column 6, line 18 - line 24 *<br>* page 8, line 3 - line 31 *<br>* page 12, line 33 - page 13, line 10 *<br>----- | 1-3,5,6,<br>10,13<br>11,12<br>4,7-9 | |

| | | TECHNICAL FIELDS SEARCHED (IPC) |
|---|---|---|
| | | G01R<br>G02B |

The present search report has been drawn up for all claims

| Place of search | Date of completion of the search | Examiner |
|---|---|---|
| Munich | 11 December 2014 | Skalla, Jörg |

**ANNEX TO THE EUROPEAN SEARCH REPORT
ON EUROPEAN PATENT APPLICATION NO.**

EP 14 17 7847

This annex lists the patent family members relating to the patent documents cited in the above-mentioned European search report.
The members are as contained in the European Patent Office EDP file on
The European Patent Office is in no way liable for these particulars which are merely given for the purpose of information.

11-12-2014

| Patent document cited in search report | | Publication date | Patent family member(s) | | Publication date |
|---|---|---|---|---|---|
| GB 2295687 | A | 05-06-1996 | NONE | | |
| US 7039266 | B1 | 02-05-2006 | US | 7039266 B1 | 02-05-2006 |
| | | | US | 7340125 B1 | 04-03-2008 |
| DE 3511020 | A1 | 03-10-1985 | DE | 3511020 A1 | 03-10-1985 |
| | | | FR | 2562359 A1 | 04-10-1985 |
| | | | IT | 1234928 B | 02-06-1992 |
| | | | JP | S60208015 A | 19-10-1985 |

EPO FORM P0459

For more details about this annex : see Official Journal of the European Patent Office, No. 12/82